# EUROPEAN PATENT SPECIFICATION

(11) **EP 1 362 577 B1**
(45) Date of publication and mention of the grant of the patent: **12.07.2006**
(21) Application number: 02010152.3
(22) Date of filing: 13.05.2002
(51) Int. Cl.: A61Q 5/10

(54) **Hair dye composition**
Haarfärbemittel
Produit pour la coloration des cheveux

(43) Date of publication of application: 19.11.2003
(73) Proprietor: KAO CORPORATION, Tokyo 103-8210 (JP)
(72) Inventor: Barbieru, Roxana, Institut für Organische Chemie, 38092 Braunschweig (DE); Grahn, Walter, 38110 Braunschweig-Waggum (DE); Matsunaga, Kenichi, 64297 Darmstadt (DE); Pratt, Dominic, 64297 Darmstadt (DE)
(74) Representative: HOFFMANN EITLE

(56) References cited:
- EP-A- 0 585 018
- EP-A- 0 661 040
- EP-A- 0 728 466
- NAPOLITANO ALESSANDRA ET AL: "Transient quinonimines and 1,4-benzothiazines of pheomelanogenesis: New pulse radiolytic and spectrophotometric evidence." FREE RADICAL BIOLOGY & MEDICINE, vol. 27, no. 5-6, 1999, pages 521-528, XP002220537 ISSN: 0891-5849

## Description

The present invention relates to a hair dye composition which has excellent hair-dyeing ability, can impart the hair with a vivid and/or natural-looking color ranging from bright yellow to chestnut, and has good fastness to shampooing.

### Description of the Background Art:

Oxidative hair dye compositions comprising oxidative dye intermediate(s) and hydrogen peroxide have heretofore been known, and it can provide excellent hair-dyeing ability, good fastness to shampooing and great variety of hair color. It is however accompanied with the drawbacks that color tone is not so vivid and color does not look natural. Furthermore, oxidative hair color compositions often cause significant degradation of hair derived from hydrogen peroxide.

On the other hand, many attempts have been carried out to provide suitable natural-looking hair color using so-called "(pseudo-)melanin" precursors and like natural pigments in hair. For example, usage of indole and indoline derivatives as "eumelanin" precursors for dyeing hair was disclosed in Japanese Laid-Open Publication Nos. Sho 62-246510, 62-270663, 63-170309, Japanese Patent Publication No.2996724(1999) etc., and usage of 1,4-dihydrobenzothiazine derivatives, catechol derivatives and combination of dihydroxybenzene and aminoethanethiol etc. as "trichochrome" or "phaeomelanin" precursors for dyeing hair was disclosed in JP-7-252118, JP-7-252120, JP-7-252121 or JP-6-183936. However, the former can provide only brown and blonde color of hair, and the latter is insufficient in hair-dyeing ability and fastness to shampooing.

### SUMMARY OF THE INVENTION

Accordingly, an object of the present invention is to provide a hair dye composition which has excellent hair-dyeing ability and good fastness to shampooing without significant damage of hair, and which provides vivid and/or suitable natural-looking hair color with covering wide range of color.

The present inventors have found that when specific 2H-1,4-benzothiazine-3-one (or 3-thione) or 2H-1,4-benzoxazine-3-one (3-thione) derivatives, or 3-substisuted-2H(4H)-benzothiazine or 3-substituted-2H-benzoxazine derivatives are used as precursors for pseudo-trichochlomes, pseudo-phaeomelanins or like pigments, a hair dye composition, which has excellent hair-dyeing ability and good fastness to shampooing, and which provides vivid and/or suitable natural-looking hair color having wide range of color variation, can be obtained, thus leading to completion of the present invention.

In one aspect of the present invention, there is thus provided a hair dye composition comprising a 2H-1,4-benzothiazine-3-one (or 3-thione) or a 2H-1,4-benzoxazine-3-one (3-thione) derivative, or 3-substisuted-2H(4H)-benzothiazine or 3-substituted-2H-benzoxazine derivative represented by formula(1), (2) or (3) or a salt thereof: 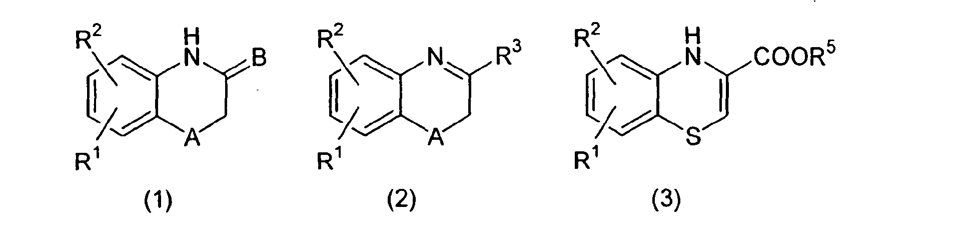 wherein each of A and B independently represents sulfur or oxygen atom; each of R¹ and R² independently represents a hydrogen atom, a C₁₋₆ alkyl group which may be substituted, a C₁₋₆ acyl group, a C₁₋₆ alkoxy group, a hydroxy group, a substituted or unsubstituted amino group, a nitro group, a C₂₋₇ alkoxycarbonyl group, a carboxy group or a carboxylate anion group having ammonium or alkali metal cation as counter ion; R³ represents a chlorine atom, a bromine atom, a iodine atom, a substituted or unsubstituted amino group, a C₁₋₆ alkoxy group, a C₁₋₆ alkylthio group, a substituted or unsubstituted phenyl group or a group represented by formula (4) or (5): 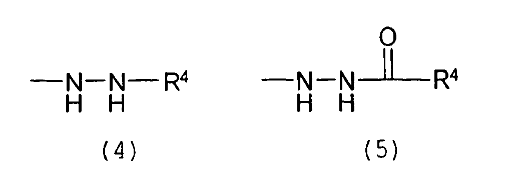 wherein R⁴ represents a hydrogen atom, a C₁₋₆ alkyl group or a substituted or unsubstituted phenyl group; R⁵ represents a hydrogen atom, a C₁₋₆ alkyl group or a substituted or unsubstituted phenyl group.

In another aspect of the present invention, there is also provided a hair dyeing method which comprises applying the above-described hair dye composition to the hair.

### BEST MODE FOR CARRYING OUT THE INVENTION

In the hair dye composition according to the present invention, compound (1), (2) and/or (3) must be used for providing both (A) excellent hair-dyeing ability and good fastness to shampooing, and (B) vivid and/or suitable natural-looking hair color simultaneously. That is to say, when the compound (1), (2) or (3) is used as precursor for pseudo-trichochlromes, pseudo-phaeomelanins or like pigments, not only the hair-dyeing ability and fastness to shampooing of the resulting hair dye composition is improved by leaps and bounds compared with the case where the precursors of (pseudo-)trichochromes, (pseudo-) phaeomelanins or like pigments mentioned in Japanese JP 7-252118, JP7-252120, JP7-252121 or 6-183936 are used, but also much wider range of color variety is obtained compared with the case where the precursors of eumelanin mentioned in JP 62-246510, JP 62-270663, JP 63-170309, Japanese Patent Publication No.2996724(1999) etc. Moreover, when the compound (1), (2) or (3) is used, hair color is vivid and/or looks much more natural compared with the case when oxidative dye intermediates are used.

The definitions for R¹, R², R³, R⁴ and R⁵ in formulas (1), (2), (3), (4) and (5) above are explained as follows in more detail.

Examples of the C₁₋₆ alkyl groups include methyl, ethyl, propyl, butyl, pentyl, hexyl, 1-methylethyl, 1-methylpropyl, 2-methylpropyl and 1,1-dimethylpropyl groups. Among them, methyl and ethyl are preferred. Examples of the subtituents for the alkyl group include one or a few hydroxy group(s), one or a few carboxy group(s), one or a few C₂₋₇ alkoxycarbonyl group(s), one or a few carboxylate anion group(s) having ammonium or alkali metal cation as counter ion, one or a few C₁₋₆ alkoxy group(s), one or more than one halogen atom(s) or one or a few amino group(s) as substituent(s). The alkyl group may have two or more than two kinds of substituents at the same time.

Examples of the alkyl groups having one or a few hydroxy group(s) include hydroxymethyl, 2-hydroxyethyl, 1-hydroxyethyl, 3-hydroxypropyl, 1,2-dihydroxyethyl and 1,2,3-trihydroxypropyl groups. Among them, 2-hydroxyethyl, 1-hydroxyethyl and 3-hydroxypropyl are preferred. Examples of the alkyl groups having one or a few carboxy group(s) include 1-carboxylmethyl, 2-carboxylethyl, 1-carboxylethyl and 1,2-dicarboxylethyl groups. Examples of the alkyl groups having one or a few alkoxycarbonyl group(s) include 1-methoxycarbonylmethyl, 1-ethoxycarbonylmethyl, 2-methoxycarbonylethyl, 2-ethoxycarbonylethyl and 1,2-di(ethoxycarbonyl)ethyl groups. Examples of the alkyl groups having one or a few carboxylate anion group(s) having alkali metal cation as counter ion include 1-(sodium carboxylate)methyl, 2-(potassium carboxylate)ethyl and 1,2-di(potassium carboxylate)ethyl groups. Examples of the alkyl groups having one or a few C₁₋₆ alkoxy group include 1,1-diethoxymethyl, 1,1-diethoxyethyl, 1,2-dimethoxyethyl and 2-ethoxyethyl groups. Examples of the halogen atoms include fluorine atom, chlorine atom, bromine atom and iodine atom. Examples of the alkyl groups having one or more than one halogen atom(s) include trifluoromethyl, pentafluoroethyl, chloromethyl, trichlorometyl, bromomethyl, 2-bromoethyl, iodomethyl and 2-iodoethyl groups. Examples of the alkyl groups having one or a few amino group(s) include aminomethyl, 2-aminoethyl, 1-aminoethyl, 3-aminopropyl, 1,2-diaminoethyl and 1,2,3,-triaminopropyl groups. Among them, 2-aminoethyl, 1-aminoethyl and 3-aminopropyl are preferred. Examples of the alkyl groups having two or more than two kinds of substituents at the same time include 2-amino-2 carboxylethyl, 2-amino-2-ethoxycarbonylethyl and 2-amino-2-(potassium carboxylate)ethyl groups.

Examples of the C₁₋₆ acyl groups include formyl, acetyl, propanoyl, butanoyl, pentanoyl, hexanoyl and 2-methylpropanoyl groups. Among them, formyl and acetyl are preferred.

Examples of the C₁₋₆ alkoxy groups include methoxy, ethoxy, propoxy, butoxy, pentyloxy, hexyloxy, 1-methylethoxy and 1,1-dimetylethoxy groups. Among them, methoxy and ethoxy are preferred.

Examples of the C₂₋₇ alkoxycarbonyl groups include methoxycarbonyl, ethoxycarbonyl, propoxycarbonyl, butoxycarbonyl, pentyloxycarbonyl, hexyloxycarbonyl and 1,1-dimetylethoxycarbonyl groups. Among them, methoxy and ethoxy are preferred.

Examples of the carboxylate anion groups having ammonium or alkali metal cation as counter ion include ammonium carboxylate, lithium carboxylate, sodium carboxylate and potassium carboxylate. Among them, sodium carboxylate and potassium carboxylate are preferred. Examples of the substituents which may substituted for amino group include C₁₋₆ alkyl group and substituted or unsubstituted phenyl group.

Examples of the substituents which may substitute on phenyl group include C₁₋₆ alkyl group, C₁₋₆ alkoxy group, hydroxy group, amino group, fluorine atom, chlorine atom, bromine atom and iodine atom.

Examples of 2H-1,4-benzothiazine-3-one (or 3-thione) or 2H-1,4-benzoxazine-3-one (3-thione) derivatives, or 3-substisuted-2H(4H)-benzothiazine or 3-substituted-2H-benzoxazine derivatives represented by formula (1), (2) or (3) include following structures.

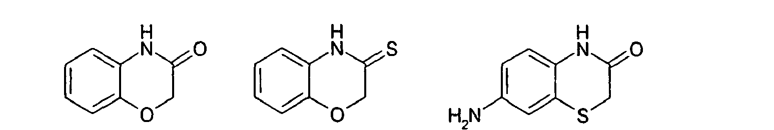

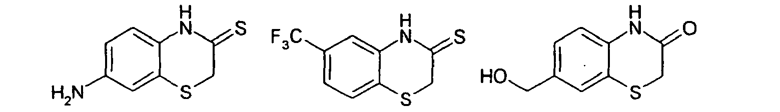

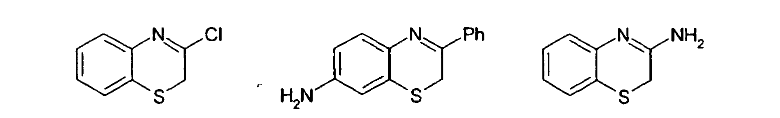

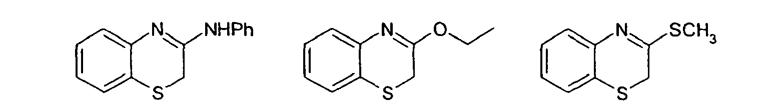

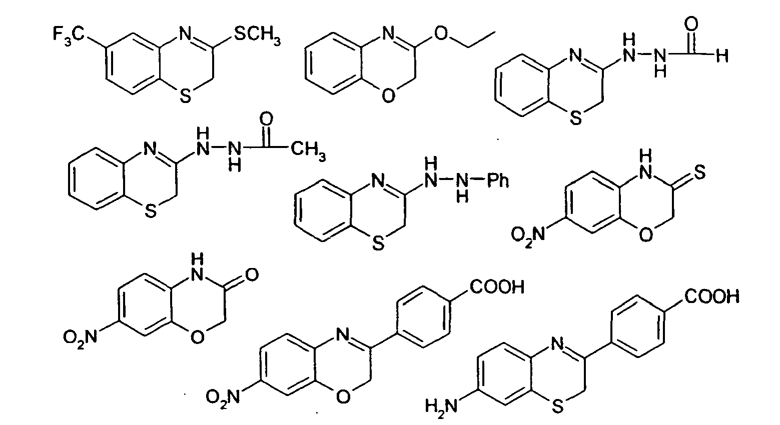

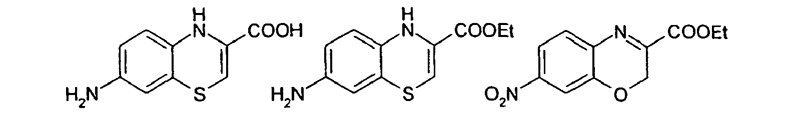

Each of the compounds of formulas (1), (2) or (3) of the present invention may be a salt of an organic or inorganic acid, for example, hydrochloric acid, sulfuric acid, phosphoric acid, acetic acid, propionic acid, lactic acid or citric acid.

Compounds of formulas (1), (2) or (3) or their salts may be used either singly or in any combination thereof and are preferably incorporated in a proportion of 0.01 to 10 wt.%, more preferably 0.1 to 5 wt.% based on the whole composition (after mixing of all the parts when a two-part or three-part composition is employed; this will apply equally hereinafter).

The 2H-1,4-benzothiazine-3-ones represented by formula(1) may be prepared, for example, in accordance with the following reaction scheme:

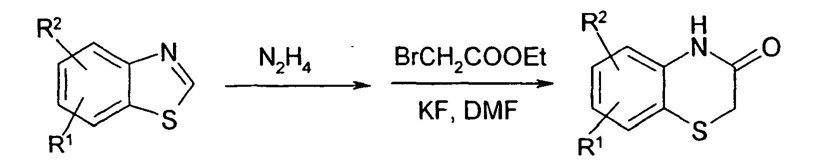

The 2H-1,4-benzoxazine-3-ones represented by formula(1) may be prepared, for example, in accordance with the following reaction scheme:

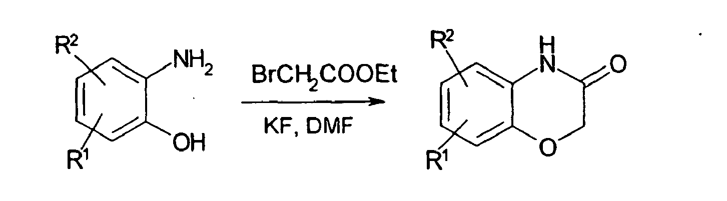

The 3-substisuted-2H-benzothiazine represented by formula(2) may be prepared, for example, in accordance with the following reaction scheme:

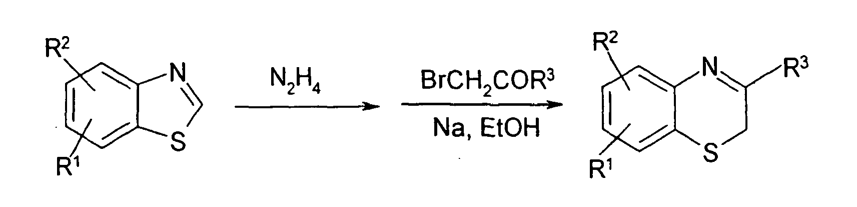

The 3-substisuted-2H-benzoxazine represented by formula(2) may be prepared, for example, in accordance with the following reaction scheme:

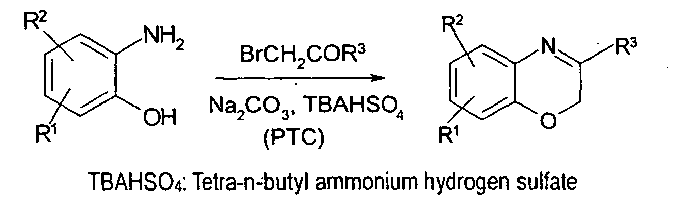

The hair dye composition of the present invention may be applied to the hair in such a manner that sufficient oxidation takes place, in the hair to provide a tinctorially effective amount of hair dyeing "pseudo-trichochromes", "pseudo-phaeomelanins" or like pigments to permanently dye the hair.

Trichochromes are polycyclic pigments generally characterized as yellow or red. Several of them are known and have been extracted from red hair and feathers under alkaline conditions.

Phaeomelanins are reddish-brown nitrogen and sulfur containing macromolecular pigments which are found in phaeomelanocytes. They are derived from tyrosinase oxidation of tyrosine and subsequent reaction with cysteine.

It is known that 3-substituted-2H-benzothiazine could be oxidized to form the following conjugated dimer (6): 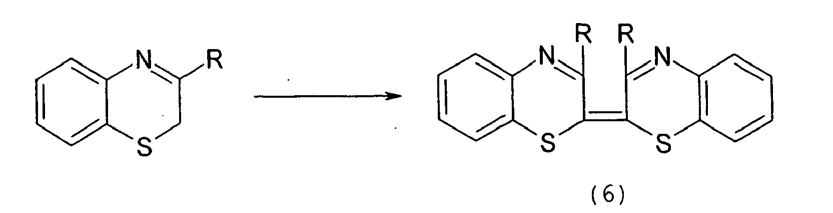 as disclosed, for example, in the literature: Angew. Chem. Internat. Edit., 13, 305(1974). The conjugated dimer has a partial structure of trichochromes, so the present inventors call this conjugated dimer as "pseudo-trichochrome".

"Pseudo-thrichochrome", "pseudo-phaeomelanin" and like compounds may be the end-product pigments, which provide natural-looking color, of this invention. It is believed that these terms and their meanings are well understood by the skilled artisan even though the exact chemical identity of some of the products is not precisely known or understood.

Moreover, compound (1), (2) or (3) or salts thereof may be used as a coupler of oxidative hair dye when it is incorporated with oxidative dye precursor(s) or as a precursor of oxidative dye when it is incorporated with oxidative dye various couplers. In these cases, too, the end products may be pigments, which provide natural-looking color.

The hair dye composition of the present invention may be (auto) oxidized by oxygen in the atmosphere, or may be oxidized by adding a chemical oxidizing agent. In the former case, oxidation may be achieved by leaving the hair in air for an appropriate time after applying the composition of the present invention and subsequent treatment of the hair by the composition having pH value of 0.5 to 4. In the latter case, oxidation may be achieved by applying the composition which comprises both the compound (1),(2) or (3) and the chemical oxidizing agent, or by applying the composition which comprises the chemical oxidizing agent after applying the composition which comprises the compound (1), (2) or (3).

Examples of particularly preferable oxidizing agents include hydrogen peroxide, persulfate, perborate, percarbonate, products obtained by adding hydrogen peroxide to urea or melamine, bromate, ferricyanide, periodate and iodate. Persulfate, perborate, percarbonate, bromate, ferricyanide, periodate and iodate include ammonium salt and salts of alkali metals, preferred sodium and potassium.

These oxidizing agents may be used either singly or in any combination thereof and are preferably incorporated in a proportion of 0.01 to 50 wt.%, more preferably 0.1 to 25 wt.% based on the whole composition.

The hair dye composition of the present invention may comprise other one or more than one oxidative dye intermediate(s), one or more than one direct dye(s) and/or one or more than one autooxidative dye precursor(s) so as to change the color.

Known precursor and/or coupler, which is generally used in oxidative hair color, may be used as oxidative dye intermediate.

Examples of precursors include p-phenylenediamine, toluene-2,5-diamine, 2-chloro-p-phenylenediamine, N-methoxyethyl-p-phenylenediamine, N,N-bis(2-hydroxyethyl)-p-phenylenediamine, 2-(2-hydroxyethyl)-p-phenylenediamine, 2,6-dimethyl-p-phenylenediamine, 4,4'-diaminodiphenylamine, 1,3-bis(N-(2-hydroxyethyl)-N-(4-aminophenyl)amino)-2-propanol, PEG-3,3,2'-p-phenylenediamine, p-aminophenol, p-methylaminophenol, 4-amino-3-methylphenol, 2-methylamino-4-aminophenol, 2-(2-hydroxymethylaminomethyl)-4-aminophenol, o-aminophenol, 2-amino-5-methylphenol, 2-amino-6-methylphenol, 2-amino-5-acetamidophenol, 3,4-diaminobenzoic acid, 5-aminosalicylic acid, 2,4,5,6-tertaaminopyrimidine, 2,5,6-triamino-4-hydroxypyrimidine, 4,5-diamino-1-(4-chlorobenzyl)pyrazol, 4,5-diamino-1-hydroxyethylprazole and their salts.

Examples of coupler include m-phenylenediamine, 2,4-diaminophenoxyethanol, 2-amino-4-(2-hydroxyethylamino)anisole, 2,4,-diamino-5-methylpenethol, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis (2,4-diaminophenoxy)propane, m-aminophenol, 5-amino-2-methylphenol, 5-(2-hydroxyethylamino)-2-methylphenol, 2,4-dichloro-3-aminophenol, 2-chloro-3-mino-6-methylphenol, 2-methyl-4-chloro-5-aminophenol, N-cyclopentyl-m-aminophenol, 2-methyl-4-methoxy-5-(2-hydroxyethyl)aminophenol, 2-methyl-4-fluoro-5-aminophenol, resorcinol, 2-methylresorcinol, 4-chlororesorcinol, 1-naphthol, 1,5-dihydroxynaphthalene, 1,7-dihydroxynaphthalene, 2,7-dihydroxynaphthalene, 2-(1-methylethyl)-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine and their salts.

These oxidative dye intermediates may be used either singly or in any combination thereof and are preferably incorporated in a proportion of 0.01 to 5 wt.%, more preferably 0.1 to 4 wt.% based on the whole composition.

Known acid dye, basic dye, disperse dye and/or reactive dye, which is generally used in oxidative hair color, may be used as direct dye.

Examples of the acid dyes include Acid Red 27 (C.I. 16185), Acid Red 51 (C.I. 45430), Acid Red 18 (C.I. 16255), Acid Red 92 (C.I. 45410), Acid Red 94 (C.I. 45440), Acid Red 52 (C.I. 45100), Acid Yellow 23 (C.I. 19140), Food Yellow 3 (C.I. 15985), Food Green 3 (C.I. 42053), Food Blue 2 (C.I. 42090), Acid Blue 74 (C.I. 73015), Pigment Red 57-1 (C.I. 15850), Acid Red 33 (C.I. 17200), Acid Red 87 (C.I. 45380), Acid Orange 7 (C.I. 15510), Acid Red 95 (C.I. 45425), Acid Yellow 73 (C.I. 45350), Acid Yellow 3 (C.I. 47005), Acid Green 25 (C.I. 61570), Solvent Green 7 (C.I. 59040), Acid Green 5 (C.I. 42095), Acid Blue 5 (C.I. 42052), Acid Blue 9 (C.I. 42090), Acid Orange 24 (C.I. 20170), Acid Violet 9 (C.I. 45190), Food Red 6 (C.I. 16155), Acid Red 26 (C.I. 16150), Food Red 1 (C.I. 14700), Acid Red 88 (C.I. 15620), Acid Orange 20 (C.I. 14600), Acid Yellow 40 (C.I. 18950), Acid Yellow 1 (C.I. 10316), Acid Yellow 36 (C.I. 13065), Acid Yellow 11 (C.I. 18820), Acid Green 1 (C.I. 10020), Acid Green 3 (C.I. 42085), Acid Violet 43 (C.I. 60730), Acid Black 1 (C.I. 20470), Acid Black 52 (C.I. 15711), Acid Blue 1 (C.I. 42045), Acid Blue 3 (C.I. 42051), Acid Blue 62 (C.I. 62045), Acid Brown 13 (C.I. 10410), Acid Green 50 (C.I. 44090), Acid Orange 3 (C.I. 10385), Acid Orange 6 (C.I. 14270), Acid Red 14 (C.I. 14720), Acid Red 35 (C.I. 18065), Acid Red 73 (C.I. 27290), Acid Red 184 (C.I. 15685), and Brilliant Black 1 (C.I. 28440).

Examples of the basic dyes include Basic Blue 7 (C.I. 42595), Basic Blue 16 (C.I. 12210), Basic Blue 22 (C.I. 61512), Basic Blue 26 (C.I. 44045), Basic Blue 99 (C.I. 56059), Basic Blue 117, Basic Violet 10 (C.I. 45170), Basic Violet 14 (C.I. 42515), Basic Brown 16 (C.I. 12250), Basic Brown 17 (C.I. 12251), Basic Red 2 (C.I. 50240), Basic Red 12 (C.I. 48070), Basic Red 22 (C.I. 11055), Basic Red 51, Basic Red 76 (C.I. 12245), Basic Red 118 (C.I. 12251:1), Basic Orange 31, Basic Yellow 28 (C.I. 48054), Basic Yellow 57 (C.I. 12719), Basic Yellow 87, Basic Black 2 (C.I. 11825) and Indolenine Yellow(C.I.48101); basic dye, which is written in Japanese Language Laid-Open Publication (PCT) Nos. Hei 8-507545, 8-501322 or 10-502946, or Japanese Laid-Open Publication No. Hei 10-194942).

Examples of the other direct dyes include 2-amino-3-nitrophenol, 2-amino-4-nitrophenol, 2-amino-5-nitrophenol, 4-amino-3-nitrophenol, 2-amino-6-chloro-4-nitrophenol, 4-hydroxypropylamino-3-nitrophenol, 3-nitro-p-hydroxyethylaminophenol, 2-nitro-p-phenylenediamine, 4-nitro-o-phenylenediamine, 4-nitro-m-phenylenediamine, 6-nitro-o-toluidine, 6-nitoro-p-toluidine, hydroxyethyl-2-nitro-p-toluidine, N,N'-bis(2-hydroxyethyl)-2-nitro-p-phenylenediamine, 2-chloro-5-nitro-N-hydroxyethyl-p-phenylenediamine, 2-nitoro-5-glycerylmethylaniline, 3-methylamino-4-nitrophenoxyethanol, N-ethyl-3-nitroPABA, picramic acid, 2-hydroxyethylpicramic acid, 4-nitrophenylaminoethylurea, Solvent Violet 13 (C.I.60725), Solvent Yellow 44 (C.I.56200), Disperse Red 17 (C.I.11210), Disperse Violet 1 (C.I.61100), Disperse Violet 4 (C.I.61105), Disperse Blue 3 (C.I.61505), Disperse Blue 7 (C.I.62500), HC Blue 2, HC Blue 8, HC Orange 1, HC Orange 2, HC Red 1, HC Red 3, HC Red 7, HC Red 8, HC Red 10, HC Red 11, HC Red 13, HC Red 16, HC Violet 2, HC Yellow 2, HC Yellow 5, HC Yellow 6, HC Yellow 7, HC Yellow 9 and HC Yellow 12.

These direct dyes may be used either singly or in any combination thereof and are preferably incorporated in a proportion of 0,001 to 5 wt.%, more preferably 0,01 to 4 wt.% based on the whole composition.

Known autooxidative dye precursor, which is generally used in oxidative hair color, also can be used. Examples of autooxidative dye precursors include 5,6-dihydroxyindole, 5,6-dihydroxyindole-2-carboxylic acid, 5,6-dihydroxyindoline and 5,6-dihydroxyindoline-2-carboxylic acid; precursors of (pseudo-)trichochrome, (pseudo-)phaeomelanin or like pigment, which are written in Japanese Laid-Open Publication Nos. Hei 7-252118, 7-252120, 7-252121 or 6-183936.

These autooxidative dye precursors may be used either singly or in any combination thereof and are preferably incorporated in a proportion of 0.01 to 5 wt.%, more preferably 0.1 to 4 wt.% based on the whole composition.

The hair dye composition of the present invention preferably has a pH value of 2 to 12, more preferably 5 to 12, particularly preferably 7.5 to 12.

The hair dye composition of the present invention may have one or more than one pH-adjusting agent(s), which are generally used as ingredients for cosmetics. Examples of pH-adjusting agents include hydrochloric acid, phosphoric acid, citric acid, glycolic acid, lactic acid, ammonia, ammonium hydrate, monoethanolamine, isopropanolamine, 2-amino-2-methylpropanol, ammonium carbonate, ammonium bicarbonate, sodium carbonate, sodium bicarbonate, potassium carbonate, potassium bicarbonate, guanidine carbonate, ammonium chloride, monoethanolamin hydrochloric acid salt, sodium hydrogenphosphate, potassium phosphate, sodium hydroxide and potassium hydroxide.

The hair dye composition of the present invention may have water and/or organic solvents as medium. Examples of organic solvents include ethanol, 2-propanol, benzyl alcohol, 2-benzyloxyethanol, propyleneglycol, 1,3-butanediol, diethyleneglycol, glycerol, 2-ethoxyethanol, 2-butoxyethanol, 2-(2-ethoxy)ethoxyethanol and 2-(2-butoxy)ethoxyethanol.

Besides the above components, components commonly used in the classical cosmetic compositions and the like, for example, surfactants, cationic polymer, oily substances, silicone derivatives, thickener, perfume bases, preservatives, ultraviolet absorbents, chelating agents, antioxidants, germicides, propellants, etc, may further be incorporated into the hair dye composition according to the present invention so as to no detrimental influence is thereby imposed on the effects of the present invention.

The hair dye composition of the present invention can be prepared in a conventional manner into a one-part composition, a two-part composition having a first-part component containing the compound represented by formulas (1), (2) or (3) or a salt thereof and a second-part component containing an oxidizing agent or a three-part composition having, in addition to these two components, a powdery oxidizing agent such as persulfate. The one-part type is applied to the hair directly and then left in air for 1 to 60 minutes. In order to increase hair-dyeing ability of the one-part type composition, treatment of the hair by the composition comprising an acidic substance, or by the composition comprising an oxidizing agent after applying the above-described one-part type composition having pH value of 7.5 to 12 for appropriate time is particularly preferred. On the other hand, the two- or three-part type is applied to the hair for 1 to 60 minutes after mixing the components upon hair dyeing.

The hair dye composition of the present invention may be preferably provided in the form of, for example, powder, cream, emulsion, gel, paste, aerosol, aerosol-foam, solution, etc.

### EXAMPLES

The following non-limiting examples are given by way of illustration only.

The compounds, which were used for evaluations, were represented by formulas (1-a), (1-b), (1-c), (2-a) and (2-b) below:

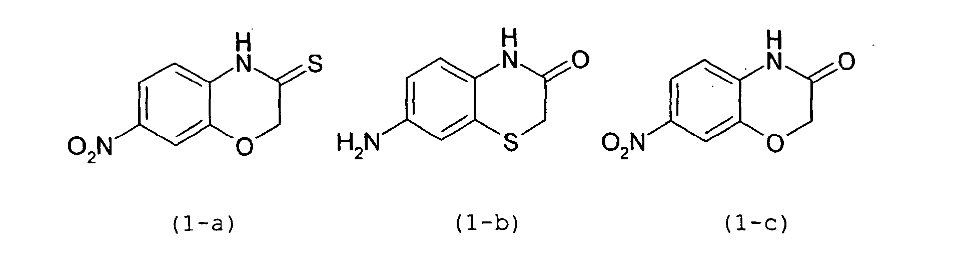

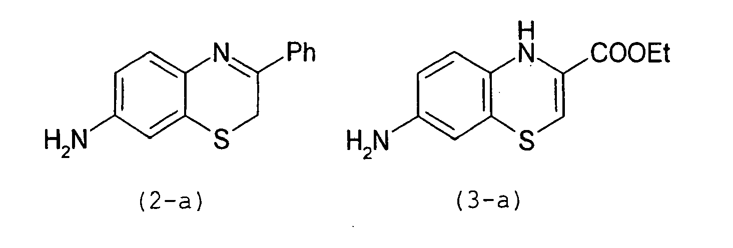

### Example 1

10mg of compound (1-a) was dissolved in 30% isopropanol in water at pH10. An untreated white goat tress was immersed in the resulting solution for 15 minutes at 50°C, and then 50 wt.% hydrogen peroxide (0.2g) was added. The tress was left for a further 5mins at 50°C before rinsing and drying. The tress was dyed a bright yellow color.
The color of hair before treatment: L=85 a=-0.3 b=12.5 The color of hair after treatment: L=81 a=-3.7 b=46.4

### Example 2

10mg of compound (1-a) was dissolved in 30 wt.% isopropanol in water at pH10. An untreated white goat tress was immersed in the resulting solution for 15 minutes at 50°C, and then 300mg potassium persulphate was added. The tress was left for 5mins at 50°C before rinsing and drying. The switch was dyed a dark yellow color.
The color of hair before treatment: L=85 a=-0.3 b=12.5
The color of hair after treatment: L=69 a=3.3 b=49

### Example 3

18mg of compound (1-b) and 14mg of alpha-Naphthol were dissolved in 30 wt.% isopropanol in water at pH 10. An untreated white goat tress was immersed in this solution for 15 minutes at 50°C, and then persulphate (300mg) was added. The tresses were left in the solution for a further 5 minutes before rinsing and drying. The tresses were dyed a dark red color.
The color of hair before treatment: L=85 a=-0.3 b=12.5
The color of hair after treatment: L=40 a=25 b=21

### Example 4

18mg of compound (1-c) and 14mg of alpha-Naphthol were dissolved in 10 wt.% isopropanol in water at pH 10. An untreated white goat tress was immersed in this solution for 15 minutes at 50°C, and then persulphate (300mg) was added. The tresses were left in the solution for a further 5 minutes before rinsing and drying. The tresses were dyed a chestnut color.
The color of hair before treatment: L=85 a=-0.3 b=12.5
The color of hair after treatment: L=48 a=15 b=31

### Example 5

10mg of compound (1-a) was dissolved in 10 wt.% isopropanol in water (10g) at pH 10. An untreated white goat tress was immersed in the solution for 15 minutes at 50°C. Then the solution was adjusted to pH 1 with HCl(aq) before the tress was removed and left in air to develop color for 5mins. After this time, the tress was rinsed and then dried to produce a bright orange coloration.
The color of hair before treatment: L=85 a=-0.3 b=12.5
The color of hair after treatment: L=72 a=12.8 b=31

### Example 6

Hair dye compositions for dyeing the hair having their corresponding compositions shown in Table 1 are prepared to evaluate them as to hair-dyeing ability, fastness to shampooing and resulting hair color.

**Table 1**

| | Invention Product (% by weight) | | | | |
|---|---|---|---|---|---|
| Component | 1 | 2 | 3 | 4 | 5 |
| Compound (1-a) | 2 | - | - | - | - |
| Compound (1-b) | - | 2 | - | - | 2 |
| Compound (2-a) | - | - | 2 | - | - |
| Compound (3-a) | - | - | - | 2 | - |
| Red Color No.227 | - | - | - | - | 0.1 |
| Ethanol | 10 | 5 | 10 | 10 | 5 |
| Propyleneglycol | 10 | - | 10 | 10 | - |
| Citric acid | - | - | - | 3 | 3 |
| Lactic acid | 3 | 3 | 3 | - | - |
| 48% NaOH aqueous solution | Adjusted to pH 3.5 | | | | |
| Hydroxyethyl cellulose | 1 | 1 | 1 | - | - |
| Xanthan gum | - | - | - | 1 | 1 |
| Purified water | Balance | Balance | Balance | Balance | Balance |

Each hair dye composition is applied to white tresses of goat hair(1g), and the tresses are then left to stand for 30 minutes at 30°C. Thereafter, the tresses are washed with water, shampooed once and dried.

The tresses are then shampooed 10 times and dried.

As a result, all compositions can provide excellent hair-dyeing ability and good fastness to shampooing, and can provide suitable reddish or yellowish natural-looking color to goat hair.

### EXAMPLE 7

Compositions having their corresponding compositions shown in Table 2 are prepared.

**Table 2**

| | Invention Product (% by weight) | | | | |
|---|---|---|---|---|---|
| Component | 6 | 7 | 8 | 9 | 10 |
| Compound (1-a) | 4 | - | - | - | - |
| Compound (1-c) | - | 4 | - | - | 4 |
| Compound (2-a) | - | - | 4 | - | - |
| Compound (3-a) | - | - | - | 4 | - |
| Red Color No.227 | - | - | - | - | 0.2 |
| Ethanol | 20 | 10 | 20 | 20 | 10 |
| Propyleneglycol | 20 | - | 20 | 20 | - |
| Citric acid | - | - | - | 0.5 | 0.5 |
| Lactic acid | 0.5 | 0.5 | 0.5 | - | - |
| 48% NaOH aqueous solution | Adjusted to pH 5.5 | | | | |
| Hydroxyethyl cellulose | 2 | 2 | 2 | - | - |
| Xanthan gum | - | - | - | 2 | 2 |
| Purified water | Balance | Balance | Balance | Balance | Balance |

Each composition is mixed with the same weight of a 10% aqueous solution of sodium periodate to thereby give a hair dye composition.

Each hair dye composition is applied to white tresses of goat hair(1g), and the tresses are then left to stand for 30 minutes at 30°C. Thereafter, the tresses are washed with water, shampooed once and dried.

The tresses are then shampooed 10 times and dried.

As a result, all compositions can provide excellent hair-dyeing ability and good fastness to shampooing, and can provide suitable reddish or yellowish natural-looking color to goat hair.

### Example 8

Compositions having their corresponding compositions shown in Table 3 are prepared.

**Table 3**

| | Invention Product (% by weight) | | | | |
|---|---|---|---|---|---|
| Component | 11 | 12 | 13 | 14 | 15 |
| Compound (1-a) | 2 | - | - | - | - |
| Compound (1-b) | - | 2 | - | - | 2 |
| Compound (2-a) | - | - | 2 | - | - |
| Compound (3-a) | - | - | - | 2 | - |
| p-Phenylenediamine | - | - | - | - | 0.2 |
| 5-Amino-2- methylphenol | - | - | - | - | 0.3 |
| Oleic acid | 10 | 10 | 10 | 10 | 10 |
| Diethanolamide oleate | 8 | 8 | 8 | 8 | 8 |
| Oleyl alcohol | 2 | 2 | 2 | 2 | 2 |
| Polyoxyethylene octadodecyl ether (20 EO) | 10 | 10 | 10 | 10 | 10 |
| 25% Ammonia | Adjusted to pH 9.5 | | | | |
| Ethanol | 15 | 15 | 15 | 15 | 15 |
| Propylene glycol | 10 | 10 | 10 | 10 | 10 |
| Ammonium chloride | 3 | 3 | 3 | 3 | 3 |
| Purified water | Balance | Balance | Balance | Balance | Balance |

Each composition is mixed with the same weight of a 6% aqueous solution of hydrogen peroxide to thereby give a hair dye composition.

Each hair dye composition is applied to white tresses of goat hair(1g), and the tresses are then left to stand for 30 minutes at 30°C. Thereafter, the tresses are washed with water, shampooed once and dried.

The tresses are then shampooed 10 times and dried.

As a result, all compositions can provide excellent hair-dyeing ability and good fastness to shampooing, and could provide suitable reddish or yellowish natural-looking color to goat hair.

## Claims

1. A hair dye composition comprising 2H-1,4-benzothiazine-3-one (or 3-thione) or 2H-1,4-benzoxazine-3-one (3-thione) derivative, or 3-substisuted-2H(4H)-benzothiazine or 3-substituted-2H-benzoxazine derivative represented by formulas (1), (2) or (3) below or salt thereof: 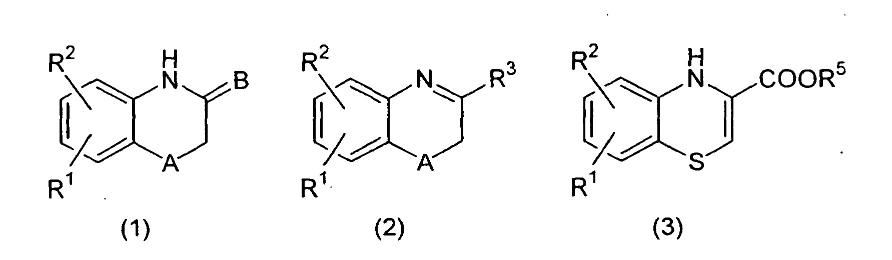 wherein each of A and B independently represents sulfur or oxygen atom; each of R¹ and R² independently represents a hydrogen atom, a C₁₋₆ alkyl group which may be substituted, a C₁₋₆ acyl group, a C₁₋₆ alkoxy group, a hydroxy group, a substituted or unsubstituted amino group, a nitro group, a C₂₋₇ alkoxycarbonyl group, a carboxy group or a carboxylate anion group having ammonium or alkali metal cation as counter ion; R³ represents a chlorine atom, a bromine atom, a iodine atom, a substituted or unsubstituted amino group, a C₁₋₆ alkoxy group, a C₁₋₆ alkylthio group, a substituted or unsubstituted phenyl group or a group represented by formula (4) or (5): 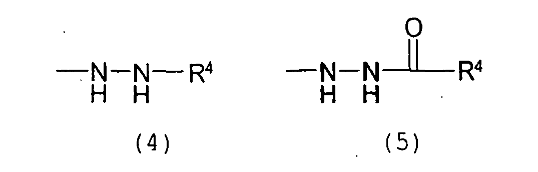 wherein R⁴ represents a hydrogen atom, a C1-6 alkyl group or a substituted or unsubstituted phenyl group; R⁵ represents a hydrogen atom, a C1-6 alkyl group or a substituted or unsubstituted phenyl group.

2. The hair dye composition according to Claim 1, which further comprises one or more than one oxidizing agent(s).

3. The hair dye composition according to Claim 1 or 2, which further comprises one or more than one oxidative dye intermediate(s), one or more than one direct dye(s) and/or one or more than one autooxidative dye precursor(s).

4. A method of dyeing the hair by applying a proper amount of the hair dye composition of Claim 1,2 or 3 to the hair.

5. A method of dyeing hair comprising applying to the hair an effective amount of the hair dye composition described in Claim 1, having pH value of 7.5 to 12, and then applying to the hair a composition comprising an acidic substance.

6. A method of dyeing hair comprising applying to the hair an effective amount of the hair dye composition described in Claim 1, having pH value of 7.5 to 12, and then applying to the hair a composition comprising an oxidizing agent.

7. Use of a composition as defined in any one of the claims 1 to 3 for dyeing the hair and for imparting the hair with a vivid and/or natural-looking colour.

## Patentansprüche

1. Haarfärbezusammensetzung, umfassend 2H-1,4-Benzothiazin-3-on- (oder 3-thion) oder 2H-1,4-Benzoxazin-3-on- (3-thion)-Derivat oder 3-substituiertes 2H(4H)-Benzothiazin- oder 3-substituiertes 2H-Benzoxazin-Derivat mit den Formeln (1), (2) oder (3) oder ein Salz davon: 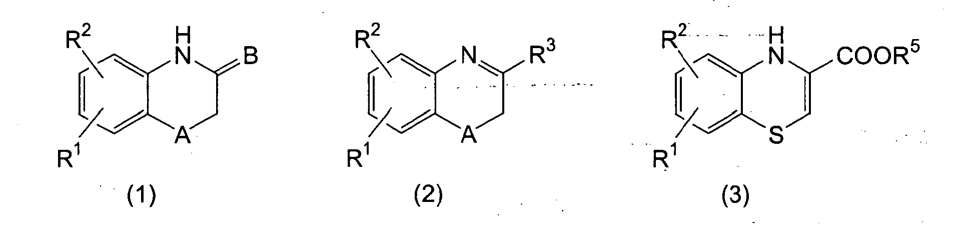 worin A und B jeweils unabhängig Schwefel- oder Sauerstoffatom sind; R¹ und R² jeweils unabhängig Wasserstoffatom, eine gegebenenfalls substituierte C₁₋₆₋Alkylgruppe, C₁₋₆-Acylgruppe, C₁₋₆-Alkoxygruppe, Hydroxygruppe, substituierte oder unsubstituierte Aminogruppe, Nitrogruppe, C₂₋₇-Alkoxycarbonylgruppe, Carboxygruppe oder Carboxylatanionengruppe mit einem Ammonium- oder Alkalimetallkation als Gegenion sind; R³ Chloratom, Bromatom, Iodatom, substituierte oder unsubstituierte Aminogruppe, C₁₋₆-Alkoxygruppe, C₁₋₆₋Alkylthiogruppe, substituierte oder unsubstituierte Phenylgruppe oder Gruppe mit der Formel (4) oder (5) sind: 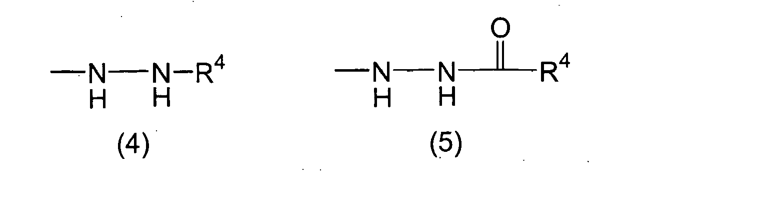 worin R⁴ ein Wasserstoffatom, eine C₁₋₆-Alkylgruppe oder substituierte oder unsubstituierte Phenylgruppe ist; R⁵ ein Wasserstoffatom, eine C₁₋₆-Alkylgruppe oder substituierte oder unsubstituierte Phenylgruppe ist.

2. Haarfärbezusammensetzung nach Anspruch 1, die weiterhin ein oder mehr als ein Oxidationsmittel umfaßt.

3. Haarfärbezusammensetzung nach Anspruch 1 oder 2, die weiterhin ein oder mehr als ein oxidative Farbstoffzwischenprodukte, einen oder mehr als einen direktziehende Farbstoffe und/oder einen oder mehr als einen autooxidativen Farbstoffvorläufer umfaßt.

4. Verfahren zum Färben von Haar durch Auftragen einer angemessenen Menge der Haarfärbezusammensetzung nach Anspruch 1, 2 oder 3 auf das Haar.

5. Verfahren zum Färben von Haar, umfassend das Auftragen einer effektiven Menge der Haarfärbezusammensetzung nach Anspruch 1 mit einem pH-Wert von 7,5 bis 12 auf das Haar und anschließendes Auftragen einer Zusammensetzung mit einer sauren Substanz auf das Haar.

6. Verfahren zum Färben von Haar, umfassend das Auftragen einer effektiven Menge der Haarfärbezusammensetzung nach Anspruch 1 mit einem pH-Wert von 7,5 bis 12 und das anschließende Auftragen einer Zusammensetzung mit einem Oxidationsmittel auf das Haar.

7. Verwendung einer Zusammensetzung nach einem der Ansprüche 1 bis 3 zum Färben des Haars und um dem Haar eine lebendige und/oder natürlich aussehende Farbe zu verleihen.

## Revendications

1. Composition pour la coloration des cheveux comprenant un dérivé de 2H-1,4-benzothiazine-3-one (ou 3-thione) ou de 2H-1,4-benzoxazine-3-one (3-thione), ou un dérivé de 3-substitué-2H(4H)-benzothiazine ou de 3-substitué-2H-benzoxazine représenté par les formules (1), (2) ou (3) ci-dessous ou un sel de celui-ci : 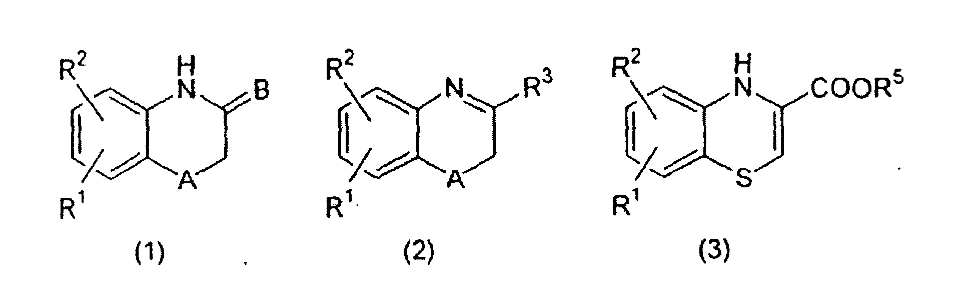 où chacun de A et B représente indépendamment un atome de soufre ou d'oxygène; chacun de R¹ et R² représente indépendamment un atome d'hydrogène, un groupe alkyle en C₁₋₆ qui peut être substitué, un groupe acyle en C₁₋₆, un groupe alcoxy en C₁₋₆, un groupe hydroxy, un groupe amino substitué ou non substitué, un groupe nitro, un groupe alcoxycarbonyle en C₂₋₇, un groupe carboxy ou un groupe anionique carboxylate ayant un cation ammonium ou de métal alcalin en tant que contre-ion; R³ représente un atome de chlore, un atome de brome, un atome de iode, un groupe amino substitué ou non substitué, un groupe alcoxy en C₁₋₆, un groupe alkylthio en C₁₋₆, un groupe phényle substitué ou non substitué ou un groupe représenté par la formule (4) ou (5) : 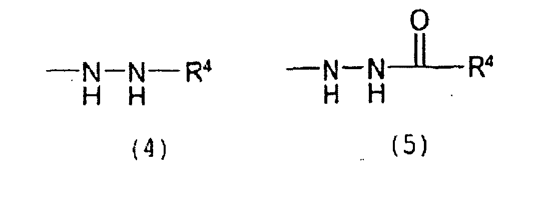 où R⁴ représente un atome d'hydrogène, un groupe alkyle en C₁₋₆ ou un groupe phényle substitué ou non substitué; R⁵ représente un atome d'hydrogène, un groupe alkyle en C₁₋₆ ou un groupe phényle substitué ou non substitué.

2. Composition pour la coloration des cheveux selon la revendication 1, qui comprend en outre un ou plus que un agent d'oxydation.

3. Composition pour la coloration des cheveux selon la revendication 1 ou 2, qui comprend en outre un ou plus que un intermédiaire de colorant oxydatif, un ou plus que un colorant direct et / ou un ou plus que un précurseur de colorant auto-oxydatif.

4. Procédé de coloration des cheveux par application d'une quantité appropriée de la composition pour la çoloration des cheveux de la revendications 1, 2 ou 3 aux cheveux.

5. Procédé de coloration des cheveux comprenant l'application aux cheveux d'une quantité efficace de la composition pour la coloration des cheveux décrite dans la revendication 1, ayant une valeur de pH de 7,5 à 12, et ensuite l'application aux cheveux d'une composition comprenant une substance acide.

6. Procédé de coloration des cheveux comprenant l'application aux cheveux d'une quantité efficace de la composition pour la coloration des cheveux décrite dans la revendication 1, ayant une valeur de pH de 7,5 à 12, et ensuite l'application aux cheveux d'une composition comprenant un agent oxydant.

7. Utilisation d'une composition telle que définie dans l'une quelconque des revendications 1 à 3 pour colorer les cheveux et pour conférer aux cheveux une couleur ayant une apparence vive et/ou naturelle.
